# EUROPÄISCHE PATENTSCHRIFT

(11) **EP 2 016 899 B1**
(45) Veröffentlichungstag und Bekanntmachung des Hinweises auf die Patenterteilung: **22.12.2010**
(21) Anmeldenummer: 07112625.4
(22) Anmeldetag: 17.07.2007
(51) Int. Cl.: A61B 5/15

(54) **Vorrichtung zum Gewinnen von Körperflüssigkeit**
Device for extracting body fluids
Dispositif destiné à l'extraction de liquides corporels

(43) Veröffentlichungstag der Anmeldung: 21.01.2009
(73) Patentinhaber: F.Hoffmann-La Roche AG, 4070 Basel (CH); Roche Diagnostics GmbH, 68305 Mannheim (DE)
(72) Erfinder: Hörauf, Christian, 68723 Oftersheim (DE); List, Hans, 64754 Hesseneck-Kailbach (DE); Zimmer, Volker, 67229 Laumersheim (DE)
(74) Vertreter: Pfiz, Thomas

(56) Entgegenhaltungen:
- WO-A-2005/107596
- DE-A1- 19 604 156
- US-A1- 2003 054 044
- US-A1- 2003 083 685
- US-A1- 2003 199 789
- US-B2- 7 223 276

## Beschreibung

Die Erfindung betrifft eine Vorrichtung zum Gewinnen von Körperflüssigkeit, insbesondere Blut, mit einem eine Spitze zum Einstechen in die Haut eines Körperteils aufweisenden Stechelement und einem mit dem Stechelement gekoppelten Aktuator für eine Vorwärts- und Rückbewegung des Stechelements, wobei das Stechelement bis zu einer gegebenenfalls einstellbaren Einstechposition in die Haut einstechbar ist. Die Erfindung betrifft weiter ein entsprechendes Verfahren.

In der Literatur sind vielfältige Vorrichtungen beschrieben, bei denen unter Elnsatz einer Einweg-Lanzette eine Hautöffnung erzeugt werden soll, um eine Flüssigkeitsprobe, insbesondere Kapillarblut möglichst schmerzarm zu entnehmen. Dies ist insbesondere bei Diabetikern für regelmäßige Blutzuckerkontrollen ein wesentlicher Gesichtspunkt. Auch in den am Markt befindlichen Geräten wurden im Laufe der Zeit immer dünnere Lanzetten eingesetzt, um den Stichschmerz so gering wie möglich zu halten. Bei einer in der PCT/EP2007/001888 offenbarten gattungsgemäßen Vorrichtung wird in diesem Zusammenhang ein Hochgeschwindigkeitsantrieb mit Stechgeschwindigkeiten von 15 m/s vorgeschlagen.

Die Offenlegungsschrift DE 196 04 156 A1 offenbart eine Schneidvorrichtung mit oszillierender Klinge zur Erzeugung eines Gewebeeinschnitts. Die aus der so erzeugten Wunde austretende Blutflüssigkeit kann dann von einem Benutzer auf einen Blutteststreifen aufgetragen werden.

Die veröffentlichte Patentanmeldung US 2003/199789 A1 offenbart ein von einem Elektromagneten angetriebenes Stechsystem, bei dem verschiedene Geschwindigkeitsprofile einstellbar sind. Zur Schmerzreduktion wird in diesem Dokument vorgeschlagen, eine Einstichgeschwindigkeit von mindestens 2 m/s zu verwenden. Ferner offenbart dieses Dokument Stechelemente, die beweglich in einer Probenaufnahmevorrichtung gelagert sind.

Ein ähnliches Stechsystem wird auch in der veröffentlichten Patentanmeldung US 2003/083685 A1 desselben Anmelders offenbart. In diesem Dokument wird ebenfalls erwähnt, dass eine geringe Rückzugsgeschwindigkeit nach dem Einstich vorteilhaft für den Erfolg der Probenentnahme ist.

Ausgehend hiervon liegt der Erfindung die Aufgabe zugrunde, eine gattungsgemäße Vorrichtung und ein damit durchführbares Verfahren im Sinne einer Schmerzreduktion zu optimieren, wobei ein Erfindungsziel auch in einer Verringerung des Herstellungsaufwandes besteht.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Der Erfindung liegt die Erkenntnis zugrunde, dass auch überraschend langsame Stechgeschwindigkeiten verwendet werden können, ohne dass hierdurch ein verstärktes Schmerzempfinden beim Benutzer verursacht wird. Dementsprechend wird erfindungsgemäß vorgeschlagen, dass der Aktuator zum Vorwärtstreiben des Stechelements auf eine Eindringgeschwindigkeit beim Eindringen in die Haut von maximal 0,7 m/s und bevorzugt zwischen 0,4 bis 0,5 m/s ausgebildet ist. Hierdurch lässt es sich erreichen, dass ein Stoppen der Spitze des Stechelements unter der Haut aufgrund der reduzierten Geschwindigkeit mit weniger Vibrationen und damit verringertem Schmerz verbunden ist. Durch die verringerte Einstich- bzw. Eindringgeschwindigkeit werden auch bei geringen Stechtiefen die erforderlichen Beschleunigungen bzw. Verzögerungen erniedrigt, wodurch sich wiederum geringere Schwingungsanregungen aller am Mechanismus beteiligten Bauteile ergeben. Dadurch wird gerade bei als Massenartikel aus Kunststoffteilen aufgebauten Handgeräten die Wahrscheinlichkeit erhöht, dass die Lanzette sich exakt auf ihrer Stechachse bewegt, ohne dass Querbewegungen überlagert werden.

Vorteilhafterweise ist der Aktuator so eingerichtet, dass das Stechelement bis zum Eindringen in die Haut beschleunigt wird. Auf diese Weise lässt sich der geräteseitige Aufwand weiter reduzieren.

Eine weitere vorteilhafte Ausgestaltung sieht vor, dass die mittlere Geschwindigkeit des Stechelements bei der Vorwärtsbewegung weniger als das 100-fache der mittleren Geschwindigkeit des Stechelements bei der Rückbewegung beträgt. Dies ist besonders wichtig bei Systemen, die in einer Ein-Schritt-Funktion sowohl Stechen als auch die Probenflüssigkeit zur Analyse aufnehmen. Das für den gesamten Bewegungsablauf angegebene Geschwindigkeitsverhältnis gilt dabei auch bei alleiniger Betrachtung des Hauteingriffs. Erfindungsgemäß ist der Aktuator ausgelegt das Stechelement bei der Rückbewegung mit einer mittleren Geschwindigkeit zwischen 1 und 10 mm/s, insbesondere zwischen 3 und 9 mm/s aus der Haut zurückzuziehen. Die mittlere Rückziehgeschwindigkeit aus der Haut heraus legt bei gegebener Stechtiefe die Sammeldauer für die Blutgewinnung fest. Hierbei sollte es für einen erfolgreichen Sammelvorgang hinreichen, wenn der Aktuator das Stechelement während einer Rückziehzeit zwischen 200 ms und 800 ms, insbesondere zwischen 250 ms und 750 ms aus der tiefsten Einstechposition bis zur Hautoberfläche zurückzieht.

Um ausreichend Blutkapillaren anzustechen und zugleich möglichst wenig Nervenenden zu verletzen, ist es günstig, wenn die tiefste Einstechposition im Bereich zwischen 1 und 2 mm, insbesondere bei etwa 1,2 mm liegt.

Für eine erhöhte Benutzerfreundlichkeit wird erfindungsgemäß vorgeschlagen, dass das Stechelement eine Sammelstruktur, insbesondere eine Kapillare zur Aufnahme der Körperflüssigkeit aufweist, so dass eine Analyse in einem Zug möglich ist.

Eine weitere Verbesserung hinsichtlich eines definierten Einstichs kann durch eine zum Positionieren des Körperteils in einem Stechbereich des Stechelements ausgebildete Positioniereinrichtung erreicht werden.

Zum Nachweis eines Inhaltsstoffs der mit dem Stechelement gewonnenen Körperflüssigkeit kann ein geeignet ausgebildetes Testelement vorgesehen sein. Solche Testelemente können auf einem optischen oder elektrochemischen Nachweis einer Zielsubstanz beruhen.

Die notwendige Ablaufsteuerung der Stechbewegung lässt sich bevorzugt dadurch realisieren, dass der Aktuator einen vorzugsweise federgetriebenen Kulissenantrieb aufweist.

Um die Stechbewegung definiert zu begrenzen, ist es vorteilhaft, wenn der Aktuator oder das Stechelement mit einem Anschlag oder Dämpfer zur Begrenzung der Vorwärtsbewegung des Stechelements zusammenwirkt.

Um Querauslenkungen möglichst zu vermeiden, sollte das Stechelement zumindest über einen Teil seiner Länge in der Bewegungsrichtung in einer Linearführung geradlinig geführt sein.

In geometrisch vorteilhafter Dimensionierung insbesondere hinsichtlich geringem Stichschmerz und hinreichender Knicksteifigkeit ist das Stechelement aus einem Runddraht mit einem Durchmesser von weniger als 0,5 mm oder aus einem Flachmaterial mit einem Dicke von weniger als 0,2 mm geformt.

Im Folgenden wird die Erfindung anhand des in der Zeichnung in schematischer Weise dargestellten Ausführungsbeispiels näher erläutert. Es zeigen
- Fig. 1: ein Handgerät mit einem automatisch angetriebenen Stechele- ment in schaubildlicher Darstellung;
- Fig. 2: ein Diagramm des Geschwindigkeitsverlaufs des Stechelements bei der Vorwärts- und Rückbewegung über dem Weg;
- Fig. 3 und 4: ein Weg- und Geschwindigkeitsdiagramm des Stechelements über der Zeit.

Die in der Zeichnung dargestellte Vorrichtung 10 umfasst einen Aktuator 12 für den Antrieb eines Stechelements 14 in einer Vorwärts- und Rückbewegung 16, wobei das Stechelement 14 bis zu einer gegebenenfalls einstellbaren Einstechposition bzw. Einstechtiefe in die Haut 18 eines Fingers 20 einstechbar ist. Eine solche Vorrichtung lässt sich bevorzugt in integrierten Stech- und Nachweissystemen für Blutzuckermessungen im Rahmen der Patienten-Selbstkontrolle einsetzen. Dabei ist der Aktuator 12 so ausgelegt, dass die Eindringgeschwindigkeit des Stechelements 14 für einen schmerzarmen Einstich auf ca. 0,4 bis 0,5 m/s begrenzt wird.

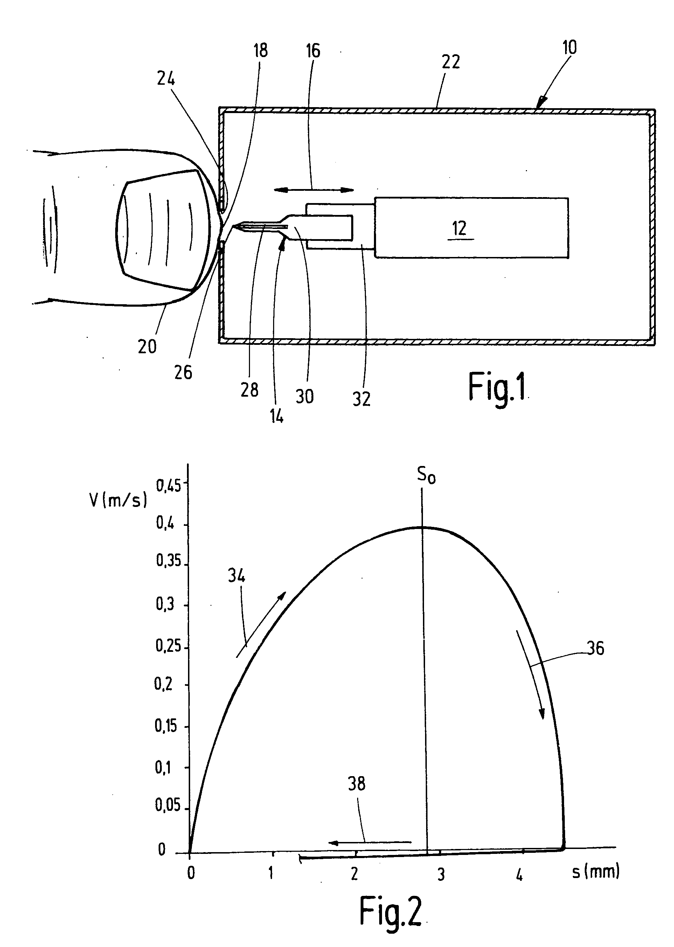

Wie in Fig. 1 veranschaulicht, kann an dem Gerätegehäuse 22 eine Öffnung 24 zur definierten Fingerpositionierung vorgesehen sein. Das Stechelement 14 lässt sich mit seiner distalen Spitze 26 in den Bereich der Öffnung 24 bewegen, um durch einen Einstich in die Haut 18 Blut und/oder Gewebeflüssigkeit zu gewinnen. Dabei kann die Fingerpositionierung gleichsam durch Melken des Fingers den Blutfluss in der Einstichwunde fördern.

Vorteilhaft erfolgt die Aufnahme der Körperflüssigkeit durch eine ggf. hydrophil beschichtete Kapillare 28 des Stechelements 14, welche mit einem analytischen Testfeld 30 fluidisch verbunden oder verbindbar ist. In dem gezeigten Beispiel ist das Stechelement 14 als Einwegteil aus einem flachen Edelstahlblech mit einer Dicke von weniger als 0,2 mm gebildet. Denkbar ist auch eine Herstellung aus einem dünnen Runddraht. Eine nicht eigens gezeigte Messeinrichtung ermöglicht beispielsweise durch eine optische Erfassung des Testfelds 30 einen Blutglukosenachweis.

Um seitliche Ausweichbewegungen zu vermeiden, kann das Stechelement 14 in einer Linearführung 32 über einen Teil seiner Länge geführt sein, so dass eine möglichst exakte geradlinige Stechbewegung erfolgt. Ein nicht gezeigter Anschlag oder Dämpfer, dessen Lage ggf. einstellbar ist, ermöglicht eine genaue Begrenzung am vorderen (distalen) Totpunkt der Stechelementbewegung.

Die hin und her gehende Bewegung 16 des Stechelements 14 lässt sich durch eine Antriebssteuerung beispielsweise mittels eines federgetriebenen Rotations-Kulissenantriebs als Aktuator 12 bewirken, wie er als solcher dem Fachmann beispielsweise aus der US 7,223,276 bekannt ist. Hierbei ist eine hohe Massenträgheit des Antriebs vorteilhaft, um das gewünschte moderate Maß an Stechdynamik auch bei hoher Federkraft zu erreichen.

Wie aus Fig. 2 ersichtlich, kann die Stechbewegung in drei Phasen unterteilt werden. In einer ersten Phase (Pfeil 34) erfolgt die Beschleunigung des Stechelements 14 außerhalb der Haut 18. Bevorzugt beschleunigt der Aktuator 12 das Stechelement 14 bis zur Auftreffposition S₀ auf der Haut 18. Auf diese Weise ist die maximale Vorwärtsgeschwindigkeit zugleich die Eindringgeschwindigkeit des Stechelements zum Zeitpunkt des Eindringens in die Haut. Denkbar ist es auch, dass das Stechelement bereits anfänglich auf eine hohe Vorwärtsgeschwindigkeit gebracht wird und bis zum Auftreffen auf der Haut schon wieder abgebremst wird.

In der zweiten Bewegungsphase (Pfeil 36) dringt die Spitze 26 des Stechelements 14 in die Haut 18 ein. Hierbei wird das Stechelement kontinuierlich (aber nicht zwingend linear) bis zum Stillstand abgebremst, wobei die Spitze 26 die tiefste Einstechposition im Bereich zwischen 1 und 2 mm Stechtiefe, gemessen von der Hautoberfläche, erreicht hat.

Bei der anschließenden Rückbewegung (Pfeil 38) wird das Stechelement 14 aus der Haut zurückgezogen. Für einen erfolgreichen Sammelvorgang ist hierbei wichtig, dass die mittlere Rückziehgeschwindigkeit ein bis zwei Größenordnungen kleiner als die mittlere Eindringgeschwindigkeit ist. Die Rückbewegung innerhalb der Haut kann auch in verschiedenen Intervallen mit unterschiedlicher Geschwindigkeit und ggf. dazwischenliegenden Pausen erfolgen. Der Schlussphase der Rückbewegung des Stechelements nach dem Verlassen der Haut bei S₀ erfolgt unabhängig vom Benutzer und lässt sich entsprechend den apparativen Randbedingen optimieren.

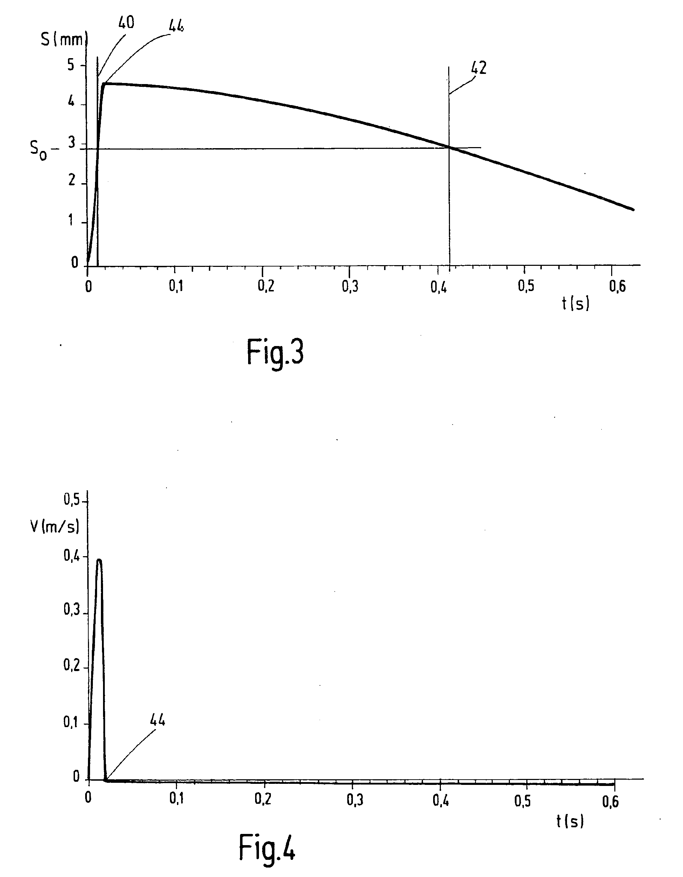

Zur weiteren Veranschaulichungen ist in den Fig. 3 und 4 das Weg- und Geschwindigkeits-Profil der Stechbewegung skalengleich über der Zeit dargestellt. In Fig. 3 kennzeichnet die horizontale Linie S₀ wieder die Hautoberfläche. Die Schnittpunkte mit der Wegkurve, markiert durch vertikale Linien 40 und 42, ergeben somit die gesamte Verweilzeit des Stechelements 14 in der Haut 18 des Körperteils 20. Während die Vorwärtsphase bis zur tiefsten Einstechposition nur wenige Millisekunden beträgt, ist von dort bis zum Verlassen der Haut bei Position 42 eine Rückziehzeit von mehreren 100 Millisekunden vorgesehen, so dass eine ausreichende Befüllung der Kapillare 28 gewährleistet ist.

Fig. 4 zeigt den zeitlichen Geschwindigkeitsverlauf, wobei das Maximum von ca. 0,4 m/s etwa im Zeitpunkt des ersten Hautkontakts, also bei Linie 40 in Fig. 3, erreicht wird. Allgemein wird als Eindringgeschwindigkeit des Stechelements die momentane Geschwindigkeit der Nadelspitze 26 im Bereich zwischen dem ersten Hautkontakt und dem tiefsten Einstechpunkt 44 definiert. Am tiefsten Einstechpunkt 44 ergibt sich ein Nulldurchgang der Geschwindigkeitskurve, wobei dann während der Rückbewegung der Betrag der mittleren Geschwindigkeit auf einem niedrigen Wert zwischen 1 und 10 mm/s liegt.

Die Messung solcher Zeitverläufe kann mit einer Hochgeschwindigkeitskamera erfolgen, wobei die Zeit mitlaufend eingeblendet wird. Für eine Bestimmung des Hautkontakts unabhängig vom Benutzer kann die durch die Geräteöffnung 24 definierte Bezugsebene gewählt werden. Gegebenenfalls kann auch ein so genannter Hautdummy das Verhalten der Haut simulieren. Hierfür eignet sich beispielsweise Silikongummi mit einer Härte von 30 Shore-A.

## Patentansprüche

1. Vorrichtung zum Gewinnen von Körperflüssigkeit, insbesondere Blut, mit einem eine Spitze (26) zum Einstechen in die Haut (18) eines Körperteils aufweisenden Stechelement (14) und einem mit dem Stechelement (14) gekoppelten Aktuator (12) für eine Vorwärts- und Rückbewegung des Stechelements (14), wobei das Stechelement (14) bis zu einer gegebenenfalls einstellbaren Einstechposition (44) in die Haut (18) einstechbar ist, **dadurch gekennzeichnet, dass** das Stechelement (14) eine Sammelstruktur (28) zur Aufnahme der Körperflüssigkeit aufweist, und dass der Aktuator (12) zum Vorwärtstreiben des Stechelements (14) auf eine Eindringgeschwindigkeit beim Eindringen in die Haut (18) von maximal 0,7 m/s ausgebildet ist und dafür ausgelegt ist, das Stechelement (14) bei der Rückbewegung mit einer mittleren Geschwindigkeit zwisehen 0,001 und 0,01 m/s zurückzuziehen.

2. Vorrichtung nach Anspruch 1, **dadurch gekennzeichnet, dass** die maximale Eindringgeschwindigkeit des Stechelements (14) bei 0,4 bis 0,5 m/s liegt.

3. Vorrichtung nach Anspruch 1 oder 2, **dadurch gekennzeichnet, dass** der Aktuator (12) das Stechelement (14) bis zum Eindringen in die Haut (18) beschleunigt.

4. Vorrichtung nach einem der Ansprüche 1 bis 3, **dadurch gekennzeichnet, dass** die mittlere Geschwindigkeit des Stechelements (14) bei der Vorwärtsbewegung weniger als das 100-fache der mittleren Geschwindigkeit des Stechelements (14) bei der Rückbewegung beträgt.

5. Vorrichtung nach einem der Ansprüche 1 bis 4, **dadurch gekennzeichnet, dass** der Aktuator (12) das Stechelement (14) bei der Rückbewegung mit einer mittleren Geschwindigkeit zwischen 3 und 9 mm/s aus der Haut (18) zurückzieht.

6. Vorrichtung nach einem der Ansprüche 1 bis 5, **dadurch gekennzeichnet, dass** der Aktuator (12) das Stechelement (14) während einer Rückziehzeit zwischen 200 ms und 800 ms, insbesondere zwischen 250 ms und 750 ms aus der tiefsten Einstechposition (44) bis zur Hautoberfläche (42) zurückzieht.

7. Vorrichtung nach einem der Ansprüche 1 bis 6, **dadurch gekennzeichnet, dass** die tiefste Einstechposition (44) im Bereich zwischen 1 und 2 mm, insbesondere bei etwa 1,2 mm liegt.

8. Vorrichtung nach einem der Ansprüche 1 bis 7, **dadurch gekennzeichnet, dass** das Stechelement (14) eine eine Kapillare zur Aufnahme der Körperflüssigkeit aufweist

9. Vorrichtung nach einem der Ansprüche 1 bis 8, **gekennzeichnet durch** eine zum Positionieren des Körperteils (20) in einem Stechbereich des Stechelements (14) ausgebildete Positioniereinrichtung (24).

10. Vorrichtung nach einem der Ansprüche 1 bis 9, **gekennzeichnet durch** ein Testelement (30), das zum Nachweis eines Inhaltsstoffs der mit dem Stechelement (14) gewonnenen Körperflüssigkeit ausgebildet ist.

11. Vorrichtung nach einem der Ansprüche 1 bis 10, **dadurch gekennzeichnet, dass** der Aktuator (12) einen vorzugsweise federgetriebenen Kulissenantrieb zur Bewegungssteuerung des Stechelements (14) aufweist.

12. Vorrichtung nach einem der Ansprüche 1 bis 11, **dadurch gekennzeichnet, dass** der Aktuator (12) oder das Stechelement (14) mit einem Anschlag oder Dämpfer zur Begrenzung der Vorwärtsbewegung des Stechelements zusammenwirkt.

13. Vorrichtung nach einem der Ansprüche 1 bis 12, **dadurch gekennzeichnet, dass** das Stechelement (14) zumindest über einen Teil in einer Linearführung (32) geradlinig geführt ist.

14. Vorrichtung nach einem der Ansprüche 1 bis 13, **dadurch gekennzeichnet, dass** das Stechelement (14) aus einem Runddraht mit einem Durchmesser von weniger als 0,5 mm geformt ist.

15. Vorrichtung nach einem der Ansprüche 1 bis 14, **dadurch gekennzeichnet, dass** das Stechelement (14) aus einem Flachmaterial mit einem Dicke von weniger als 0,2 mm gebildet ist.

## Claims

1. Device for obtaining body fluid, in particular blood, comprising a lancing element (14) having a tip (26) for puncturing the skin (18) of a body part and an actuator (12) coupled with the lancing element (14) for a forward and backward movement of the lancing element (14), where the lancing element (14) can be inserted into the skin (18) up to an optionally adjustable puncture position (44), **characterized in that** the lancing element (14) has a collecting structure (28) for taking up the body fluid, and that the actuator (12) is designed to drive the lancing element (14) forwards to a penetration speed of at most 0.7 m/s when it penetrates the skin (18) and is designed to retract the lancing element (14) during the return movement at an average speed between 0,001 and 0,01 m/s.

2. Device according to claim 1, **characterized in that** the maximum penetration speed of the lancing element (14) is 0.4 to 0.5 m/s.

3. Device according to claim 1 or 2, **characterized in that** the actuator (12) accelerates the lancing element (14) until it penetrates the skin (18).

4. Device according to one of the claims 1 to 3, **characterized in that** the average speed of the lancing element (14) during the forward movement is less than 100-times the average speed of the lancing element (14) during the return movement.

5. Device according to one of the claims 1 to 4, **characterized in that** the actuator (12) retracts the lancing element (14) from the skin (18) during the return movement at an average speed between 3 and 9 mm/s.

6. Device according to one of the claims 1 to 5, **characterized in that** the actuator (12) retracts the lancing element (14) from the deepest puncture position (44) to the skin surface (42) during a retraction period of between 200 ms and 800 ms and in particular between 250 ms and 750 ms.

7. Device according to one of the claims 1 to 6, **characterized in that** the deepest puncture position (44) is in a range between 1 and 2 mm and in particular at about 1.2 mm.

8. Device according to one of the claims 1 to 7, **characterized in that** the lancing element (14) has a capillary for taking up the body fluid.

9. Device according to one of the claims 1 to 8, **characterized by** a positioning device (24) designed to position the body part (20) in a lancing area of the lancing element (14).

10. Device according to one of the claims 1 to 9, **characterized by** a test element (30) which is designed to detect a substance contained in the body fluid obtained with the lancing element (14).

11. Device according to one of the claims 1 to 10, **characterized in that that** the actuator (12) has a preferably spring-driven sliding gate drive to control the movements of the lancing element (14).

12. Device according to one of the claims 1 to 11, **characterized in that** the actuator (12) or the lancing element (14) interact with a stop or damping device to limit the forward movement of the lancing element.

13. Device according to one of the claims 1 to 12, **characterized in that** at least part of the lancing element (14) is guided in a straight line in a linear guide (32).

14. Device according to one of the claims 1 to 13, **characterized in that** the lancing element (14) is formed from a round wire having a diameter of less than 0.5 mm.

15. Device according to one of the claims 1 to 14, **characterized in that** the lancing element (14) is formed from a flat material having a thickness of less than 0.2 mm.

## Revendications

1. Dispositif destiné à extraire du liquide corporel, en particulier du sang, comprenant un élément piqueur (14) doté d'une pointe (26), destinée à piquer la peau (18) d'une partie du corps, et un actionneur (12) couplé à l'élément piqueur (14) pour un mouvement d'avancement et de retour de l'élément piqueur (14), l'élément piqueur (14) étant susceptible de piquer la peau (18) jusqu'à une position de piqûre (44) le cas échéant réglable, **caractérisé en ce que** l'élément piqueur (14) comporte une structure collectrice (28) pour recueillir le liquide corporel et **en ce que** l'actionneur (12), pour faire avancer l'élément piqueur (14), est conçu pour une vitesse de pénétration au moment de pénétrer dans la peau (18) d'au maximum 0,7 m/s et est conçu pour faire reculer l'élément piqueur (14) lors du mouvement de retour avec une vitesse moyenne comprise entre 0,001 et 0,01 m/s.

2. Dispositif selon la revendication 1, **caractérisé en ce que** la vitesse maximale de pénétration de l'élément piqueur (14) est de 0,4 à 0,5 m/s.

3. Dispositif selon la revendication 1 ou 2, **caractérisé en ce que** l'actionneur (12) accélère l'élément piqueur (14) jusqu'à la pénétration dans la peau.

4. Dispositif selon l'une des revendications 1 à 3, **caractérisé en ce que** la vitesse moyenne de l'élément piqueur (14) lors du mouvement d'avancement est inférieure à 100 fois la vitesse moyenne de l'élément piqueur (14) lors du mouvement de retour.

5. Dispositif selon l'une des revendications 1 à 4, **caractérisé en ce que** l'actionneur (12), lors du mouvement de retour, retire l'élément piqueur (14) de la peau (18) avec une vitesse moyenne comprise entre 3 et 9 mm/s.

6. Dispositif selon l'une des revendications 1 à 5, **caractérisé en ce que** l'actionneur (12), pendant un temps de retrait compris entre 200 ms et 800 ms, en particulier entre 250 ms et 750 ms, retire l'élément piqueur (14) de la plus profonde position de piqûre (44) jusqu'à la surface de la peau (42).

7. Dispositif selon l'une des revendications 1 à 6, **caractérisé en ce que** la plus profonde position de piqûre (44) est comprise entre 1 et 2 mm, en particulier de l'ordre de 1,2 mm.

8. Dispositif selon l'une des revendications 1 à 7, **caractérisé en ce que** l'élément piqueur (14) comporte un tube capillaire pour recueillir le liquide corporel.

9. Dispositif selon l'une des revendications 1 à 8, **caractérisé par** un dispositif de position (24), conçu pour positionner la partie du corps (20) dans une zone de piqûre de l'élément piqueur (14).

10. Dispositif selon l'une des revendications 1 à 9, **caractérisé par** un élément de test (30), conçu pour déceler un composant du liquide corporel extrait à l'aide de l'élément piqueur (14).

11. Dispositif selon l'une des revendications 1 à 10, **caractérisé en ce que** l'actionneur (12) comporte une commande à coulisse, de préférence à ressort, pour commander les mouvements de l'élément piqueur (14).

12. Dispositif selon l'une des revendications 1 à 11, **caractérisé en ce que** l'actionneur (12) ou l'élément piqueur (14) coopère avec une butée ou amortisseur, destiné à limiter le mouvement d'avancement de l'élément piqueur.

13. Dispositif selon l'une des revendications 1 à 12, **caractérisé en ce que** l'élément piqueur (14), est guidé au moins sur une partie de façon rectiligne dans un guidage linéaire (32).

14. Dispositif selon l'une des revendications 1 à 13, **caractérisé en ce que** l'élément piqueur (14) est formé à partir d'un fil de section circulaire de diamètre inférieur à 0,5 mm.

15. Dispositif selon l'une des revendications 1 à 14, **caractérisé en ce que** l'élément piqueur (14) est formé à partir d'un matériau plat d'épaisseur inférieure à 0,2 mm.
